# EUROPEAN PATENT SPECIFICATION

(11) **EP 4 263 775 B1**
(45) Date of publication and mention of the grant of the patent: **01.01.2025**
(21) Application number: 21819520.4
(22) Date of filing: 08.12.2021
(51) Int. Cl.: C11D 3/33, C07C 227/40, C07C 229/16

(54) **PROCESS FOR MAKING A SOLID ALKALI METAL SALT OF AN AMINOCARBOXYLATE COMPLEXING AGENT**
VERFAHREN ZUR HERSTELLUNG EINES FESTEN ALKALIMETALLSALZES EINES AMINOCARBOXYLAT-KOMPLEXBILDNERS
PROCÉDÉ DE FABRICATION D'UN SEL DE MÉTAL ALCALIN SOLIDE D'UN AGENT COMPLEXANT D'AMINOCARBOXYLATE

(30) Priority: 17.12.2020 EP 20215142
(43) Date of publication of application: 25.10.2023
(73) Proprietor: BASF SE, 67056 Ludwigshafen am Rhein (DE)
(72) Inventor: MUELLER, Michael Klemens, 67056 Ludwigshafen (DE); VOGES, Matthias, 67056 Ludwigshafen (DE); HARTMANN, Markus, 67056 Ludwigshafen (DE); JAEKEL, Frank, 67056 Ludwigshafen am Rhein (DE); SCHMIDT, Astrid, 67056 Ludwigshafen (DE)
(74) Representative: BASF IP Association
(86) International application number: PCT/EP2021/084836
(87) International publication number: WO 2022/128692

(56) References cited:
- EP-A1- 3 472 294
- WO-A1-2020/094480
- US-A1- 2014 155 646
- US-A1- 2020 095 189
- BUSS-SMS-CANZLER: "Drying Technology Buss-SMS-Canzler 2 Buss-SMS-Canzler Core Competence Drying", 31 December 2012 (2012-12-31), pages 1 - 16, XP055808297, Retrieved from the Internet <URL:https://www.sms-vt.com/uploads/media/Buss-SMS-Canzler_Drying_Technology_03.pdf> [retrieved on 20210527]

## Description

The present invention is related to a process for making a solid alkali metal salt (A) of an aminocarboxylate complexing agent, said process comprising the steps of
(a) providing a 40 to 80% by weight aqueous solution or slurry comprising a salt (A),
(b) optionally, heating said slurry or solution to a temperature in the range of from 50 to 150°C,
(c) introducing the slurry or solution from step (a) or, if applicable, step (b) into an essentially horizontal cylindrical drying apparatus containing a stirring element that rotates around an essentially horizontal axis and that is charged with solid particles of salt (A), wherein the pressure in the cylindrical drying apparatus is in the range from 100 to 600 mbar abs, and
(d) removing most of the water by evaporation,
(e) removing solid salt (A) as granule.

Chelating agents of the aminocarboxylate type such as methyl glycine diacetic acid (MGDA) and glutamic acid diacetic acid (GLDA) and their respective alkali metal salts are useful sequestrants for alkaline earth metal ions such as Ca²⁺ and Mg²⁺. A lot of aminocarboxylates show good biodegradability and are thus environmentally friendly. For that reason, they are recommended and used for various purposes such as laundry detergents and for automatic dishwashing (ADW) formulations, in particular for so-called phosphate-free laundry detergents and phosphate-free ADW formulations.

Depending on the type of product - liquid home care and fabric care products versus solid home care and fabric care products - and the manufacturing process of solid home care and fabric care products care product manufacturers may either prefer to handle solutions of aminocarboxylates or solid aminocarboxylates, for example joint spray drying or solid mixing. Powders and granules of aminocarboxylates may be shipped economically due to their high active ingredient content that goes along with low water content. Therefore, convenient processes for providing granules are still of great commercial interest.

In EP 0 845 456 A, a process to make crystalline salts of MGDA is disclosed. A water-containing super-cooled melt of MGDA is seeded with MGDA powder and introduced into a vessel under kneading. The process has its shortcomings, though, because it is tedious to remove the crystals from the crystallization vessel and a high energy input is required.

In WO 2009/103822, a process is disclosed in which slurries are granulated that have a certain solids content, with a gas inlet temperature of 120°C or less. In WO 2012/168739, a process is disclosed wherein slurries of complexing agents are spray-dried under non-agglomerating conditions.

Both processes have their shortcomings. A low gas inlet temperature requires highly concentrated slurries or a huge amount of gas per unit of granule. A process using non-agglomerating conditions provides for powders only.

In WO 2019/007944, a process is disclosed wherein MGDA-Na₃ is transferred into a so-called crystal type III by a combination of heating and low pressure-treatment.

US 2014/155646 A1 discloses a process for making MGDA or GLDA salts, wherein a first evaporation step takes place in a Sambay thin-film evaporator operating under vacuum, and a second evaporation step takes place in a thin-film contact dryer such as high-speed paddle dryers from various manufacturers, for example Turbodryer from Vomm, horizontal thin-film dryers from Buss, short-path evaporators from 3V Cogeim or horizontal centrifugal dryer-reactors from VRV. This process provides salts with a degree of crystallinity of ≧30%.

It is desired to provide chelating agents in solid form that are less hygroscopic and give no or little raise to yellowing upon contact with percarbonate. It is therefore an objective of the present invention to provide chelating agents in solid form that are less hygroscopic and give no or little raise to yellowing upon contact with percarbonate, and it is an objective of the present invention to provide a process for manufacturing such chelating agents in solid form. In addition, the resulting solid should exhibit excellent flowability even after storage.

Accordingly, the process as defined at the outset has been found, hereinafter also referred to as inventive process or process according to the present invention. The inventive process comprises several steps that may be referred to as step (a), step (b) etc. and that will be explained in more detail below. Step (b) is optional.

The inventive process is a process for making a solid alkali metal salt (A) of an aminocarboxylate complexing agent, hereinafter in brief also referred to as "solid salt (A)", and the inventive process comprises the following steps:
(a) providing a 40 to 80% by weight aqueous solution or slurry comprising a salt (A), preferably 45 to 75% by weight.
(b) optionally, heating said slurry or solution to a temperature in the range of from 50 to 150°C,
(c) introducing the slurry or solution from step (b) or (a), respectively, into an essentially horizontal cylindrical drying apparatus containing a stirring element that rotates around an essentially horizontal axis and that is charged with solid particles of salt (A), and
(d) removing most of the water by evaporation,
(e) removing solid salt (A) as granule.

In step (a), an aqueous solution or slurry of an aminocarboxylate complexing agent (A) is provided, hereinafter also referred to as "salt (A)". In this context, alkali metal salts are selected from lithium salts, sodium salts, potassium salts, rubidium salts, and cesium salts and combinations of at least two of the foregoing, with potassium salts being preferred and sodium salts being more preferred.

Examples of aminocarboxylate complexing agents are iminodisuccinates, and diacetates of amino acids, especially alanine, glutamic acid, and aspartic acid.

Preferably, salt (A) is selected from methylglycine diacetic acid (MGDA) and glutamic acid diacetic acid (GLDA). Even more preferably, salt (A) is selected from MGDA.

Salts (A) may refer to fully neutralized aminocarboxylate complexing agents (A) and to partially neutralized aminocarboxylate complexing agents (A).

In one embodiment of the present invention, salt (A) is selected from compounds according to general formula (I)

[CH₃-CH(COO)-N(CH₂-COO)₂]M₃₋ₓHₓ (I)

wherein
M is selected from alkali metal cations, same or different, preferably K or Na or combinations thereof, and even more preferably Na, and
x is in the range of from zero to 1.0, preferably zero to 0.5.

In any way, aqueous solutions or slurries of salt (A) may bear a cation other than alkali metal. It is thus possible that minor amounts, such as 0.01 to 5 mol-% of total MGDA, respectively, bear alkali earth metal cations such as Mg²⁺ or Ca²⁺, or an Fe²⁺ or Fe³⁺ cation.

As mentioned above, in step (a) an aqueous solution or slurry of salt (A) is provided. Aqueous solutions are defined herein as solutions with no solid particles detectable by visual inspection. Aqueous solutions may contain minor amounts of organic solvent that is or are miscible with water, for example ethanol, 1,2-propylenglycol, ethylene glycol, for example in a volume ration water: organic solvent 5:1 to 100:1. Preferably, however, aqueous solutions provided in step (a) do not contain detectable amounts of organic solvent.

Slurries, on the other hand, contain solid particles of salt (A) that are detectable by visual inspection. Slurries are preferred. Aqueous slurries of salt (A) may be obtained by several ways:
Embodiment (a1): An aqueous solution of salt (A) is provided, for example concentrated or even supersaturated, and a powder of salt (A) is added, for example an amorphous powder obtained by spray drying.
Embodiment (a2): An aqueous concentrated or even supersaturated solution of salt (A) is provided, and upon storage, a slurry of a precipitate is formed.
Embodiment (a3): An aqueous solution of salt (A) is provided and further concentrated to form a slurried crystalline precipitate, for example as evaporation crystallization.
Embodiment (a4): An aqueous solution of salt (A) is provided, for example concentrated or even supersaturated, and crystals of salt (A) are added, for example obtained by crystallization or by adding crystals from a crystalline granule, with or without milling. The term "crystalline" includes materials that are crystalline to 65% or more, determined by X-ray diffraction.
Embodiment (a5): An aqueous slurry of salt (A) is provided, for example obtained according to (a2) or (a3) or (a4), and wet-milled, for example with 100 to 10,000 rpm. High values of 1,000 or more rpm may be achieved with an ultra-turrax.
Embodiments (a2), (a3), (a4), and (a5) are preferred.

In one version of embodiment (a4), the amount of added crystals is preferably 0.5 to 2 % by weight of the total amount of salt (A) in the so obtained slurry. Optionally, from the slurry so obtained water, may be removed within one to seven hours, preferably in two to five hours and even more preferably in three to four hours, for example as evaporation crystallization.

Salts (A) are selected from the racemic mixtures, the D-isomers and the L-isomers, and from mixtures of the D- and L-isomers other than the racemic mixtures. Preferably, salt (A) is selected from the racemic mixture and from mixtures containing in the range of from 51 to 95 mole-% of the L-isomer, the balance being D-isomer. Particularly preferred are solutions of salt (A) being selected from the racemic mixture and mixtures of the enantiomers with predominantly the L-enantiomer with an ee value in the range of from 0.1% or from 0.5% to 35%. Other particularly preferred embodiments are racemic mixtures.

In one embodiment of the present invention, aqueous solutions or slurries of salt (A) may contain one or more impurities that may result from the synthesis of the respective salt (A). Such impurities may be selected from propionic acid, lactic acid, alanine, nitrilotriacetic acid (NTA) or the like and their respective alkali metal salts. Such impurities are usually present in minor amounts. "Minor amounts" in this context refer to a total of 0.1 to 5% by weight, referring to salt (A), preferably up to 2.5% by weight. In the context of the present invention, such minor amounts are neglected when determining the composition of granule made according to the inventive process.

In one embodiment of the present invention, aqueous solutions or slurries of salt (A) contain one or more inorganic salts, for example alkali metal hydroxide, alkali metal (bi)carbonate, alkali metal formate, or the like, for example in amounts of 0.5 to 10 % by weight, referring to salt (A).

In an optional step (b), the solution or preferably slurry from step (a) is heated to a temperature in the range of from 50 to110°C, for example for a duration in the range of from 10 minutes to 8 hours, preferred are 2 to 4 hours. A temperature higher than 100°C is possible due to the high content of salts and other ingredients.

In step (c) the slurry or solution from step (a) or, if applicable, step (b) is introduced into an essentially horizontal cylindrical drying apparatus containing a stirring element that rotates around an essentially horizontal axis and that is charged with solid particles of salt (A). In this context, cylinders are understood to be right cylinders but to also include conical frustums with a difference of the radius of one base and the other base to no larger than 10%, preferably no larger than 5% and more preferably no larger than 1%.

In the context with cylindrical drying apparatuses, the term "essentially horizontal" does not only include exactly horizontal cylindrical drying apparatuses but also slightly tilted drying apparatuses, for example up to 15° tilt angle.

Said cylindrical horizontal drying apparatus used in steps (c) and (d) preferably have a bigger length compared to the diameter of their base, for example by a factor of 1.5 to 10, preferably of from 2 to 7. The terms length and base refer to the cylindrical drying apparatus in a horizontal form. The rotation axis reaches from one base to the other.

In one embodiment of the present invention, the overall length of such cylindrical drying apparatuses is in the range of from 4 to 12 meter, 6 to 10 meter are preferred.

In one embodiment of the present invention, the diameter of the base of such cylindrical drying apparatuses is in the range of from 0.8 to 3 meter, 1.2 to 2.3 meter are preferred.

Said cylindrical drying apparatus contains a stirring element that rotates around an essentially horizontal axis, for example hooks and counterhooks, paddle mixers or plough share mixers, paddle mixers being preferred. Such paddle mixers preferably include a shaft that is heated in order to avoid cooling zones in the middle of said cylindrical drying apparatus.

In one embodiment of the present invention, said cylindrical drying apparatus may include more stirring elements, for example baffles.

Said cylindrical drying apparatus has one or more heated surfaces. The heating is performed for example with a double jacket, electrical or half-coil pipes. In a preferred embodiment, the inner cylindrical walls, the circular side walls, the shaft, and the paddles are heated. Possible heat carriers are electrical, oil, water or steam, oil and steam are preferred.

In one embodiment of the present invention, said cylindrical drying apparatus contains heating elements at the wall, preferably at the outer wall so an indirect heating may be achieved, for example half-coil pipes. In other embodiments, said cylindrical drying apparatus is a double wall cylinder, with oil or hot steam being the heat carrier.

In one embodiment of the present invention, the cylindrical drying apparatus is a continuous fluidization technology dryer ("CFT"). Such dryers are known and commercially available from Buss SMS Canzler GmbH.

Said slurry or solution is introduced into a cylindrical drying apparatus that is charged with solid particles of salt (A), for example to a level of filling in the range of from 50 to 90%, preferably 60 to 80%. Such solid particles of salt (A) are preferably spheroidal, and their average diameter is similar to the desired diameter of the granule to be manufactured, for example 75 to 95% of the specified diameter.

Said introducing may be performed through one or more dosing lances or nozzles, for example single-fluid nozzles and two-fluid nozzles, two-fluid nozzles being preferred. In such embodiments, the first fluid is the solution or slurry according to step (a) or (b), if applicable, the second fluid is compressed gas, for example with a pressure of 1.1 to 7 bar. Said compressed hot gas may have a temperature in the range of from 100 to 250°C, preferably 125 to 220°C. The introduction of solution or slurry from step (a) or, if applicable, step (b) preferably occurs from the top of the cylindrical drying apparatus.

In one embodiment, the feed is introduced using one or more extruders. If one or more extruders are applied, fines from an optional milling and sieving step can be introduced trough this extruder as well.

Step (c) may be performed throughout step (d), continuously or intermittently, especially in embodiments wherein the inventive process is carried out as continuous process.

In one embodiment of the present invention, the solution or slurry according to step (a) or (b), if applicable, is heated to 115 to 150°C and under elevated pressure and then introduced into said cylindrical drying apparatus in accordance to step (c), preferably with a one-component nozzle. Preferably, the solution or slurry according to step (a) or (b), if applicable, is heated to 70 to 107°C under ambient pressure and then introduced into said cylindrical drying apparatus in accordance to step (c), preferably with a one-component nozzle.

In step (d), most of the water is removed by evaporation. Solid salt (A) may, when removed, see step (e), still contain residual moisture, for example 1 to 20% by weight, preferably 7 to 14% by weight, referring to the solids content, determined by drying at 200°C for 1 hour at a Thermo balance, for example the METTLER-TOLEDO HX204.

The pressure during step (d) is in the range of from 100 to 600 mbar abs in the cylindrical drying apparatus. For technical reasons, 100 to 200 mbar abs are preferred.

In one embodiment of the present invention, the temperature of the gas inside the cylindrical vessel is in the range of from 70 to 150°C, preferably 80 to 120 °C and even more preferably 100 to 115°C.

In one embodiment of the present invention, during step (d) the rotation speed of the stirring element is in the range of from 10 to 300 revolutions per minute (rpm), preferred in production scale 30 to 180, even more preferred 30 to 80. Depending on the diameter of the cylindrical vessel and of the stirring element, e.g., the stirring paddle or stirring blade, that results in a tip velocity of 0.8 to 20 m/s.

The torque to be introduced during steps (c) and (d) is considerably lower than in corresponding processes according to EP 0 845 456. In addition, the inventive process has a comparably low energy consumption compared to conventional spray drying or spray granulation processes.

Step (e) includes removing solid salt (A) as granule. Preferably, solid salt (A) is removed from the bottom of the cylindrical vessel, preferably at a location opposite to the location were slurry or solution is introduced in accordance to step (c).

Step (e) may be performed throughout step (d), continuously or intermittently, especially in embodiments wherein the inventive process is carried out as continuous process. Said removal is preferably performed by removing salt (A) that passes a weir in the drying apparatus.

In one embodiment of the present invention, a sieving step is performed subsequently to step (e). Said - optional - sieving step may be used for removing fines and lumps from the granule. Said lumps to be separated off are particles that may have a minimum particle diameter of 150% of the desired diameter, for example, 1,500 µm to 5 mm or even more. Fines may have an average diameter in the range from particles diameter in the range of from 1 to 150 µm. The desired diameter corresponds to the respective specified diameter of potential customers.

In one embodiment, lumps are milled down and then put on the before mentioned sieves and sieved again. Only the fines are returned into the drying vessel, for example by using them as solids in embodiments (a3) or (a4). The sequence of mill and sieves can be applied interchangeably.

In one embodiment, the lumps are milled down and returned into the drying vessel together with the fines.

Further - optional - steps are, e.g., rounding steps and cooling steps. In one embodiment, the resulting granules are rounded to increase bulk density. In one embodiment, an additional downstream dying step (post drying) is applied. In one embodiment, the granules are cooled down to improve storage behavior.

By the inventive process, a granule with excellent properties may be obtained. The term "granule" in the context of the present invention refers to particulate materials that are solids at ambient temperature and that preferably may have an average particle diameter (D50) in the range of from 0.1 mm to 2 mm, preferably 0.4 mm to 1.25 mm, even more preferably 400 µm to 1 mm. The average particle diameter of inventive granules can be determined, e.g., by optical or preferably by sieving methods. Sieves employed may have a mesh in the range of from 60 to 3,000 µm.

Especially, granules obtained according the inventive process are less hygroscopic and give no or little raise to yellowing upon contact with percarbonate. They can therefore be used favourably in cleaners such as laundry detergents and automatic dishwashing detergents, for example in the form of powders or tabs, in combination with bleaching agents such as peroxides and percarbonates.

Said granule may essentially contain only salt (A) and moisture. In other embodiments, said granule may contain one or more additives, for example up to 20% by weight, preferably 7 to 14% by weight, referring to salt (A). Examples of additives are silicates, especially sodium silicate, citrates, especially sodium citrate, polyvinylalcohol, and (co)polymers other than polyvinylalcohol, hereinafter also referred to as (co)polymers (B).

(Co)polymer (B) is selected from polymers (B) of (meth)acrylic acid and of copolymers (B) of (meth)acrylic acid, preferably of acrylic acid, partially or fully neutralized with alkali. In the context of the present invention, copolymers (B) are those in which at least 50 mol-% of the comonomers are (meth)acrylic acid, preferably at least 75 mol-%, even more preferably 80 to 99 mol-%.

Suitable comonomers for copolymers (B) are ethylenically unsaturated compounds, such as styrene, isobutene, ethylene, α-olefins such as propylene, 1-butylene, 1-hexene, and ethylenically unsaturated dicarboxylic acids and their alkali metal salty and anhydrides such as but not limited to maleic acid, fumaric acid, itaconic acid disodium maleate, disodium fumarate, itaconic anhydride, and especially maleic anhydride. Further examples of suitable comonomers are C₁-C₄-alkyl esters of (meth)acrylic acid, for example methyl acrylate, methyl methacrylate, ethyl acrylate, ethyl methacrylate, n-butyl acrylate.

In one embodiment of the present invention, (co)polymer (B) is selected from copolymers of (meth)acrylic acid and a comonomer bearing at least one sulfonic acid group per molecule. Comonomers bearing at least one sulfonic acid group per molecule may be incorporated into copolymer (B) as free acid or least partially neutralized with alkali. Particularly preferred sulfonic-acid-group-containing comonomers are 1-acrylamido-1-propanesulfonic acid, 2-acrylamido-2-propanesulfonic acid, 2-acrylamido-2-methylpropanesulfonic acid (AMPS), 2-methacrylamido-2-methylpropanesulfonic acid, 3-methacrylamido-2-hydroxypropanesulfonic acid, allylsulfonic acid, methallylsulfonic acid, allyloxybenzenesulfonic acid, methallyloxyben-zenesulfonic acid, 2-hydroxy-3-(2-propenyloxy)propanesulfonic acid, 2-methyl-2-propene-1-sulfonic acid, styrenesulfonic acid, vinylsulfonic acid, 3-sulfopropyl acrylate, 2-sulfoethyl methacrylate, 3-sulfopropyl methacrylate, sulfomethacrylamide, sulfomethylmethacrylamide, and salts of said acids, such as the sodium salts, potassium salts or ammonium salts thereof.

Copolymers (B) may be selected from random copolymers, alternating copolymers, block copolymers and graft copolymers, alternating copolymers and especially random copolymers being preferred.

Useful copolymers (B) are, for example, random copolymers of acrylic acid and methacrylic acid, random copolymers of acrylic acid and maleic anhydride, ternary random copolymers of acrylic acid, methacrylic acid and maleic anhydride, random or block copolymers of acrylic acid and styrene, random copolymers of acrylic acid and methyl acrylate. More preferred are homopolymers of methacrylic acid. Even more preferred are homopolymers of acrylic acid.

(Co)polymer (B) may constitute straight-chain or branched molecules. Branching in this context will be when at least one repeating unit of such polymer (B) is not part of the main chain but forms a branch or part of a branch. Preferably, polymer (B) is not cross-linked.

In one embodiment of the present invention, (co)polymer (B) has an average molecular weight M_{w} in the range of from 1,200 to 30,000 g/mol, preferably from 2,500 to 15,000 g/mol and even more preferably from 3,000 to 10,000 g/mol, determined by gel permeation chromatography (GPC) and referring to the respective free acid.

(Co)polymer (B) is partially neutralized with alkali, for example with lithium or potassium or sodium or combinations of at least two of the forgoing, especially with sodium. For example, in the range of from 10 to 95 mol-% of the carboxyl groups of (co)polymer (B) may be neutralized with alkali, especially with sodium, preferably in the range from 20 to 70 mole-% of the carboxyl groups, even more preferred in the range of from 25 to 60 mole-%.

In one embodiment of the present invention, copolymer (B) is selected from a combination of at least one polyacrylic acid and at least one copolymer of (meth)acrylic acid and a comonomer bearing at least one sulfonic acid group per molecule, both polymers being partially neutralized with alkali.

In one embodiment of the present invention, (co)polymer (B) is selected from sodium salts of polyacrylic acid with an average molecular weight M_{w} in the range of from 1,200 to 30,000 g/mol, preferably from 2,500 to 15,000 g/mol and even more preferably from 3,000 to 10,000 g/mol, determined by gel permeation chromatography (GPC) and referring to the respective free acid.

If addition of a (co)polymer (B) is desired, said (co)polymer (B) may be added before or after performing step (b) and before step (c), or in a separate unit during step (c).

Said additive or additives - if present - is/are then homogeneously dispersed within the particles of granule of salt (A).

A further aspect of the present invention is related to solid alkali metal salts (A), hereinafter also referred to as inventive salts, of a mixture of enantiomers of the trisodium salt of MGDA, MGDA-Na₃, wherein said inventive salt comprises in the range of from 10 to 75% by weight of a crystalline material whose lattice has a monoclinic space group P2₁. Said crystalline material is also referred to as crystalline fraction, and its form is also referred to as "Form IV".

Inventive salts may be racemic or contain mixtures of enantiomers of MGDA-Na₃ wherein the majority is the L-enantiomer. In one embodiment of the present invention, inventive salts are predominantly L-MGDA-Na₃ with an ee value in the range of from 0.1 to 35%, preferably 5 to 35%.

In one embodiment of the present invention, Form IV is characterized by the following reflections when analyzed by powder diffraction with Swiss Light Source, wavelength 0.7 Å:

| d [Å] | Relative intensity I [%] |
|---|---|
| 10.79 | 100 |
| 3.79 | 21 |
| 2.81 | 17 |
| 11.00 | 17 |
| 3.67 | 16 |
| 3.87 | 16 |
| 5.55 | 13 |
| 7.74 | 10 |
| 5.26 | 9 |
| 4.18 | 9 |

| | |
|---|---|
| d: lattice spacing, Å: Angstrom. The relative intensity refers to the largest peak at 10.79 Å. | |

The table shows the ten reflections with the highest intensity. They are characteristic for Form IV. The respective X-ray powder diffraction pattern can be indexed using a monoclinic space group P2₁ of the following lattice parameters:
β: 129.635 (1) °
lattice parameter a: 21.876(8) Å
lattice parameter b: 8.709(3) Å
lattice parameter c: 14.286(5) Å

The invention is further illustrated by working examples.

The following drying apparatuses were used:
Apparatus 1: a horizontal cylindrical dryer, volume 5 liter with double-jacket oil heating, length: 35 cm, diameter of the base: 14 cm, and paddle mixer with 12 stirring blades.
Apparatus 2: a horizontal dryer, volume 6 liter, with double-jacket steam heating, length: 41.3 cm, diameter of the base: 13.4, and paddle mixer with 11 stirring blades.
rpm: revolutions per minute. Percentages are % weight unless specifically noted otherwise.
Salt (A.1): MGDA-Na₃ (ee. of 21%, determined by HPLC with penicillamine as chiral modification agent), provided as 40% by weight aqueous solution, pH: 13.

### Example

### I Manufacture of inventive granules - Granule Gr.1

### I.1 Manufacture of solution SL.1

Step (a.1): A vessel was charged with 6 kg of an aqueous solution of (A.1) (40 % by weight), SL.1.

Step (b.1): The solution SL.1 was stirred and heated to 70°C and then subjected to granulation.

### I.2 Granulation of solution SL.1

Step (c.1): Apparatus 1 was charged with 1.5 I of granule MGDA-Na₃, average diameter (D50) 700 µm (about 1.1 kg), as bed. The granule was stirred with 240 rpm. The walls of Apparatus 1 were heated to a temperature of 140°C. The pressure was adjusted to 300mbar, abs.

Step (d.1): A feed of 500 g/hour of SL.1 was introduced into Apparatus and the majority of the water of the feed is evaporated. The bed level Apparatus 1 was adjusted with a weir, additional formed granules diluted and replace the initial bed, and left the dryer over the weir through a ball valve into a sample bottle, which was also evacuated to 300 mbar, abs. After collection of 500 mL granule, the ball valve was closed, vacuum in the sample bottle was broken with air, and the granules were removed from the sample bottle. Then, vacuum of 300mbar, abs was adjusted again in the sample bottle and the ball valve was opened, to collect new granules. Step (e.1): After collection of 3 kg, a steady state was reached, and the granules so obtained are the inventive granules Gr.1.

### II Manufacture of inventive granules - Granule Gr.2

### II.1 Manufacture of spray slurry SL.2

Step (a.2): A vessel was charged with 4.18 kg of an aqueous solution of (A.1) (40 % by weight) and 1.82 kg granules MGDA-Na₃, average diameter, (D50) 700 µm (81% A.1). An aqueous slurry SL.2 was obtained.

Step (b.2): The slurry SL.2 was stirred and heated to 70°C and then subjected to granulation.

### II.2 Granulation of slurry SL.2

Step (c.2) was like step (c.1).

Step (d.2) was like (d.1), but with 1000 g/h feed. Inventive granule Gr.2 was obtained.

Step (e.2): Granule Gr.2 was collected in a way like Gr.1. More granule could be collected per hour.

### III Manufacture of inventive granules - Granule Gr.3

### 111.1 Manufacture of slurry SL.3

Steps (a.3) was like (a.2).

Step (b.3): The slurry SL.3 was stirred and heated to 70°C and then stirred at 70°C for 3 hours .

### III.2 Granulation of slurry SL.3

The protocol of II.2 was followed with SL.3. Granule Gr.3 was obtained.

### IV Manufacture of inventive granules - Granule Gr.4

### IV.1 Manufacture of slurry SL.4

Step (a.4): A crystallizer with stirrer and baffles was charged with 5.0 kg of a 40% by weight solution of salt (A.1). The solution was concentrated to 47% by weight by evaporation at 70°C and 220 mbar.

Step (b.4): The solution was maintained at 70°C with stirring at 500 rpm. Then, under stirring, 10 g (0.5 % of salt (A.1) in solution) of crystalline MGDA-Na₃, of which 85% was orthorhombic MGDA-Na₃, were added to the solution in the crystallizer. The resultant slurry was stirred for 20 minutes and subsequently was concentrated to 53% by weight by vacuum evaporation at 70°C and 220 mbar in 4 hours. The resultant slurry was stirred at 70°C with 500 rpm for 3 hours. Slurry SL.4 was obtained.

### IV.2 Granulation of slurry SL.4

The protocol of II.2 was followed with SL.4. Granule Gr.4 was obtained.

### V. Comparative example:

Manufacture of a comparative granule by fluidized bed spray granulation

The spray granulation was performed in a vertical cylindrical drying apparatus with zig-zag air classifier, commercially available as Glatt Lab Systems with Vario 3 Insert.
Nm³: cubic meter at normal conditions - 1 atm, 23°C

Step (a.5): A vessel was charged with 15 kg of an aqueous solution of (A.1) (40 % by weight), SL.5.

Step (b.5): The solution SL.5 was stirred and heated to 70°C and then subjected to fluidized bed spray granulation.

Granulation of spray solution 5 in a fluidized bed spray granulation (Glatt lab systems with Vario 3 insert):
The vertical cylindrical vessel was charged with 0.9 kg of solid MGDA-Na₃ spherical particles, diameter 350 to 1000 µm, and 600 g of milled MGDA-Na₃ particles. An amount of 200 Nm³/h of air with a temperature of 170°C was blown from the bottom. A fluidized bed of MGDA-Na₃ particles was obtained. The above liquor SL.5 was introduced by spraying 7 kg of SL.1 (temperature of solution: 70°C) per hour into the fluidized bed from the bottom through a two-fluid nozzle, absolute gas pressure in the nozzle: 5 bar. Granules were formed, and the bed temperature, which corresponds to the surface temperature of the solids in the fluidized bed, was 98 to 101°C.

Particles which are large (heavy) enough fell through the zigzag air classifier into a sample bottle. The smaller (lighter) granules were blown through the recycle back into the fluidized bed by the air classifier. The particles in the sample bottle were classified by a 1000µm screen. The granules below 1000µm are the value fraction. The granules over 1000µm are the overs (oversized particles). The overs and if necessary, some granules of the value fraction, are milled down using a Kinematica Polymix PX-MFL 90D at 4000 rpm, 2 mm mesh. The milled granules were introduced into the fluidized bed through the milled product recycle and served as seeds for new granules.

After 90 minutes of spray granulating a steady state was reached. Then, the granule C-Gr.5 was collected.

### VI. Evaluation of Gr.1 to Gr.4 and C-Gr.5

### Percarbonate stability:

An amount of 10g of granule (inventive or comparative) was mixed with 5 g of sodium percarbonate Na₂CO₃·1.5 H₂O₂, commercially available from Evonik (tradename Q30). The mixture so obtained was filled into a cell culture flask (or tissue culture flask) closed with a ventilated closure (German: Schraubverschlußkappe mit Membran) and stored under air at 35°C and 70% humidity. Before storing as well as after 12 days, after 19 days and after 26 days, the diffuse reflection was determined as remission and measured with a spectrometer for determining the whiteness, manufacturer: Konica Minolta Spectrophotometer CM-2600d, measuring b-value at wavelength of 360nm - 740nm. A high diffusion reflection corresponds to a high yellowing of the sample. The diffuse reflection values obtained are summarized in table below:
The results are summarized in Table 1:

**Table 1: Yellowing results of granules made according to the inventive process and a comparative granule**

| b value | Gr.1 | Gr.2 | Gr.3 | Gr.4 | C-Gr.5 |
|---|---|---|---|---|---|
| b-value before storage | 3.09 | 2.48 | 1.72 | 1.86 | 4.31 |
| Δ b after 12 days | 2.19 | 2.35 | 1.76 | 1.62 | 13.57 |
| Δ b after 19 days | 12.79 | 3.69 | 5.12 | 3.86 | 4.42 |
| Δ b after 26 days | 5.57 | 3.76 | 2.49 | 1.30 | 5.00 |
| b-value total yellowing | 23.64 | 12.28 | 11.09 | 8.64 | 27.30 |

The values of Δ b refer to the respective previous measurements.

### Flowability tests:

An amount of 5 g of respective granule was stored in a petri dish under air at 35°C and 70% humidity for 168 hours.

After 24 h, after 48 hours and after 168 hours the flowability was visual determined. The petri dish was smoothly shaken manually.

The rating was determined visually by flowability grades e.g.: from 0 = good flowability up to 4 = granule is dissolved in water.

**Table 2: Flowability test results of inventive granules and a comparative granule**

| grade | Gr.1 | Gr.2 | Gr.3 | Gr.4 | C-Gr.5 |
|---|---|---|---|---|---|
| after 24 hours | 2.0 | 0 | 0 | 0 | 2.0 |
| after 48 hours | 2.0 | 0 | 0.5 | 0.5 | 2.0 |
| after 168 hours | 2.0 | 0 | 0.5 | 0.5 | 3.0 |

### VII. Powder X-ray diffraction analysis of Gr.4

A powder X-ray analysis was performed on Gr.4. Data were collected at the Material Science beamline at the Swiss Light Source (SLS) using a wavelength of 0.7Å. A share of 48% by weight was Form IV.

The ee value was 21%.

The following reflections were obtained

| d [Å] | Relative intensity I [%] |
|---|---|
| 10.79 | 100 |
| 3.79 | 21 |
| 2.81 | 17 |
| 11.00 | 17 |
| 3.67 | 16 |
| 3.87 | 16 |
| 5.55 | 13 |
| 7.74 | 10 |
| 5.26 | 9 |
| 4.18 | 9 |

| | |
|---|---|
| d: lattice spacing. Å: Angstrom. The relative intensity refers to the largest peak at 10.79 Å. The ten reflections with the highest intensity are characteristic for Form IV obtained from granule Gr.4. | |

Further reflections were found as follows:

| d [Å] | Relative intensity I [%] |
|---|---|
| 6.83 | 8 |
| 4.61 | 8 |
| 5.40 | 8 |
| 3.30 | 8 |
| 4.05 | 8 |
| 4.35 | 7 |
| 3.97 | 7 |
| 4.18 | 6 |

The respective X-ray powder diffraction pattern was indexed using a monoclinic space group P2₁ of the following lattice parameters:
β: 129.635 (1) °
lattice parameter a: 21.876(8) Å
lattice parameter b: 8.709(3) Å
lattice parameter c: 14.286(5) Å

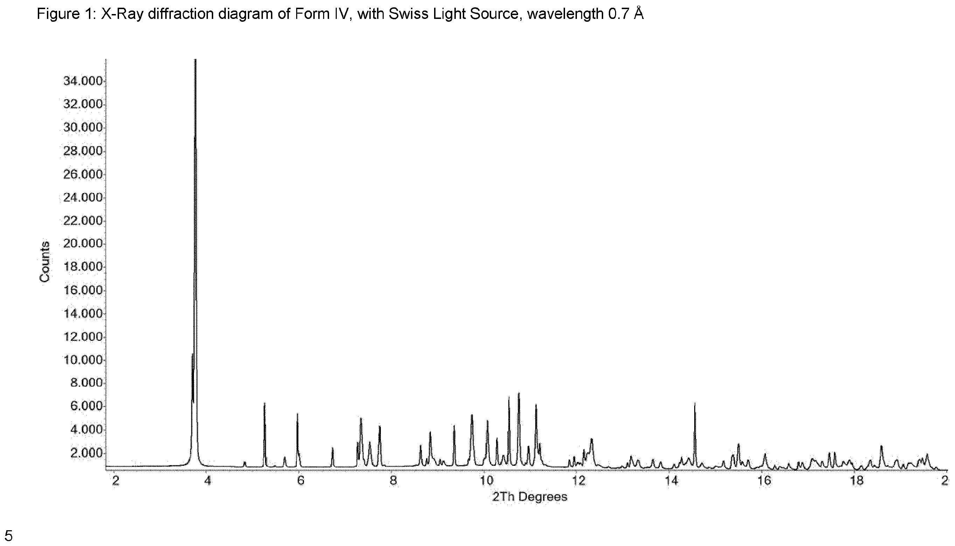

The diffraction diagram is shown in Figure 1.

## Claims

1. Process for making a solid alkali metal salt (A) of an aminocarboxylate complexing agent, said process comprising the steps of
(a) providing a 40 to 80% by weight aqueous solution or slurry comprising a salt (A),
(b) optionally, heating said slurry or solution to a temperature in the range of from 50 to 150°C,
(c) introducing the slurry or solution from step (a) or, if applicable, step (b) into an essentially horizontal cylindrical drying apparatus containing a stirring element that rotates around an essentially horizontal axis and that is charged with solid particles of salt (A), wherein the pressure in the cylindrical drying apparatus is in the range from 100 to 600 mbar abs, and
(d) removing most of the water by evaporation,
(e) removing solid salt (A) as granule.

2. Process according to claim 1 wherein the drying apparatus contains heating elements at the wall.

3. Process according to any of the preceding claims wherein said aminocarboxylate complexing agent is selected from methylglycine diacetic acid (MGDA) and glutamic acid diacetic acid (GLDA).

4. Process according to any of the preceding claims wherein salt (A) is selected from compounds according to general formula (I)
[CH₃-CH(COO)-N(CH₂-COO)₂]M₃₋ₓHₓ (I)
wherein
M is selected from alkali metal cations, same or different, and
x in formula is in the range of from zero to 1.0.

5. Process according to any of the preceding claims wherein methylglycine diacetate (MGDA) alkali metal salt (A) is selected from the racemic mixture and mixtures of the enantiomers with predominantly the L-enantiomer with an ee value in the range of from 0.1 to 35%.

6. Process according to any of the preceding claims wherein salt (A) withdrawn in step (e) has a moisture content in the range of from 1 to 20% by weight.

7. Process according to any of the preceding claims wherein the solution or slurry provided in step (a) contains at least one (co)polymer (B) selected from (co)polymers of (meth)acrylic acid and polyethylenimines, non-substituted or substituted with alkoxy groups or CH₂COOH groups that may be neutralized with alkali metal.

8. Process according to any of the preceding claims wherein a sieving step is performed subsequently to step (e).

9. Process according to any of the preceding claims wherein the cylindrical drying apparatus is a continuous fluidization technology dryer.

10. Process according to any of the preceding claims wherein the rotation speed of the stirring element is in the range of from 10 to 300 revolutions per minute.

## Patentansprüche

1. Verfahren zum Herstellen eines festen Alkalimetallsalzes (A) eines Aminocarboxylat-Komplexbildners, wobei das Verfahren die folgenden Schritte umfasst:
(a) Bereitstellen einer 40 bis 80 gew.-%igen wässrigen Lösung oder Aufschlämmung, umfassend ein Salz (A),
(b) gegebenenfalls Erhitzen der Aufschlämmung oder Lösung auf eine Temperatur im Bereich von 50 bis 150°C,
(c) Eintragen der Aufschlämmung oder Lösung aus Schritt (a) oder bzw. Schritt (b) in einen im Wesentlichen horizontalen zylindrischen Trocknungsapparat, der ein um eine im Wesentlichen horizontale Achse rotierendes Rührelement enthält und mit festen Partikeln von Salz (A) beladen ist, wobei der Druck in dem zylindrischen Trocknungsapparat im Bereich von 100 bis 600 mbar abs liegt und
(d) Abdampfen des größten Teils des Wassers,
(e) Austragen des festen Salzes (A) als Granulat.

2. Verfahren nach Anspruch 1, wobei der Trocknungsapparat Heizelemente an der Wand enthält.

3. Verfahren nach einem der vorhergehenden Ansprüche, wobei der Aminocarboxylat-Komplexbildner aus Methylglycindiessigsäure (MGDA = methylglycine diacetic acid) und Glutaminsäurediessigsäure (GLDA = glutamic acid diacetic acid) ausgewählt ist.

4. Verfahren nach einem der vorhergehenden Ansprüche, wobei Salz (A) aus Verbindungen gemäß der allgemeinen Formel (I) ausgewählt wird:
[CH₃-CH(COO)-N(CH₂-COO)₂]M₃₋ₓHₓ (I)
wobei
M aus Alkalimetallkationen, die gleich oder verschieden sind, ausgewählt ist und
x in der Formel im Bereich von null bis 1,0 liegt.

5. Verfahren nach einem der vorhergehenden Ansprüche, wobei das Methylglycindiessigsäure(MGDA)-Alkalimetallsalz (A) aus dem racemischen Gemisch und Gemischen der Enantiomere, die hauptsächlich aus dem L-Enantiomer bestehen, mit einem ee-Wert im Bereich von 0,1 bis 35 % ausgewählt wird.

6. Verfahren nach einem der vorhergehenden Ansprüche, wobei das in Schritt (e) entnommene Salz (A) einen Feuchtigkeitsgehalt im Bereich von 1 bis 20 Gew.-% aufweist.

7. Verfahren nach einem der vorhergehenden Ansprüche, wobei die in Schritt (a) bereitgestellte Lösung oder Aufschlämmung mindestens ein (Co)polymer (B) enthält, das aus (Co)polymeren von (Meth)acrylsäure und Polyethyleniminen, die unsubstituiert oder mit Alkoxygruppen oder CH₂COOH-Gruppen, die mit Alkalimetall neutralisiert sein können, substituiert sind, ausgewählt ist.

8. Verfahren nach einem der vorhergehenden Ansprüche, wobei ein Siebschritt im Anschluss an Schritt (e) durchgeführt wird.

9. Verfahren nach einem der vorhergehenden Ansprüche, wobei es sich bei dem zylindrischen Trocknungsapparat um einen kontinuierlichen Wirbelschichttrockner handelt.

10. Verfahren nach einem der vorhergehenden Ansprüche, wobei die Rotationsgeschwindigkeit des Rührelements im Bereich von 10 bis 300 Umdrehungen pro Minute liegt.

## Revendications

1. Procédé de préparation d'un sel solide de métal alcalin (A) d'un agent complexant aminocarboxylate, ledit procédé comprenant les étapes de
(a) fourniture d'une solution ou suspension aqueuse à 40 à 80 % en poids comprenant un sel (A),
(b) éventuellement, chauffage de ladite suspension ou solution jusqu'à une température dans la plage allant de 50 à 150 °C,
(c) introduction de la suspension ou solution de l'étape
(a) ou, le cas échéant, de l'étape (b) dans un appareil de séchage cylindrique essentiellement horizontal contenant un élément agitateur qui tourne autour d'un axe essentiellement horizontal et qui est chargé de particules solides de sel (A), la pression dans l'appareil de séchage cylindrique étant dans la plage de 100 à 600 mbars abs, et
(d) élimination de la majeure partie de l'eau par évaporation,
(e) élimination de sel solide (A) en tant que granule.

2. Procédé selon la revendication 1, dans lequel l'appareil de séchage contient des éléments chauffants au niveau de la paroi.

3. Procédé selon l'une quelconque des revendications précédentes, dans lequel ledit agent complexant aminocarboxylate est choisi parmi l'acide méthylglycine diacétique (MGDA) et l'acide glutamique diacétique (GLDA) .

4. Procédé selon l'une quelconque des revendications précédentes, dans lequel le sel (A) est choisi parmi des composés selon la formule générale (I)
[CH₃-CH(COO)-N(CH₂-COO)₂]M₃₋ₓHₓ (I)
dans laquelle
M est choisi parmi les cations de métaux alcalins, identiques ou différents, et
x dans la formule est dans la plage allant de zéro à 1,0.

5. Procédé selon l'une quelconque des revendications précédentes, dans lequel le sel de métal alcalin (A) du diacétate de méthylglycine (MGDA) est choisi parmi le mélange racémique et des mélanges des énantiomères comportant principalement l'énantiomère L avec une valeur de ee dans la plage allant de 0,1 à 35 %.

6. Procédé selon l'une quelconque des revendications précédentes, dans lequel le sel (A) retiré dans l'étape (e) a une teneur en humidité dans la plage allant de 1 à 20 % en poids.

7. Procédé selon l'une quelconque des revendications précédentes, dans lequel la solution ou suspension fournie dans l'étape (a) contient au moins un (co)polymère (B) choisi parmi les (co)polymères d'acide (méth)acrylique et les polyéthylèneimines, non substitués ou substitués par des groupes alcoxy ou des groupes CH₂COOH qui peuvent être neutralisés avec un métal alcalin.

8. Procédé selon l'une quelconque des revendications précédentes, dans lequel une étape de tamisage est réalisée après l'étape (e).

9. Procédé selon l'une quelconque des revendications précédentes, dans lequel l'appareil de séchage cylindrique est un sécheur à technologie de fluidisation continue.

10. Procédé selon l'une quelconque des revendications précédentes, dans lequel la vitesse de rotation de l'élément d'agitation est dans la plage allant de 10 à 300 tours par minute.
